# EUROPEAN PATENT SPECIFICATION

(11) **EP 1 742 590 B1**
(45) Date of publication and mention of the grant of the patent: **13.06.2018**
(21) Application number: 05779895.1
(22) Date of filing: 05.04.2005
(51) Int. Cl.: A61B 18/14, A61B 18/00

(54) **DEVICE FOR TREATING TISSUE**
VORRICHTUNG ZUR GEWEBEBEHANDLUNG
DISPOSITIF DESTINE AU TRAITEMENT DES TISSUS

(30) Priority: 05.04.2004 US 559568 P; 05.04.2004 US 559495 P
(43) Date of publication of application: 17.01.2007
(60) Divisional application: 18159696.6
(73) Proprietor: The General Hospital Corporation, Boston, MA 02114 (US)
(72) Inventor: GANZ, Robert A., Minnetonka, MN 55305 (US); ZELLICKSON, Brian D., Minneapolis, MN 55416 (US)
(74) Representative: Maiwald Patent- und Rechtsanwaltsgesellschaft mbH
(86) International application number: PCT/US2005/011683
(87) International publication number: WO 2005/096980

(56) References cited:
- WO-A2-03/068046
- WO-A2-2005/039689
- US-A- 5 697 909
- US-A- 5 976 129
- US-A1- 2002 120 260
- US-A1- 2003 125 728
- US-A1- 2004 039 429
- US-B1- 6 277 116
- US-B1- 6 416 514

## Description

### FIELD OF THE INVENTION

The invention relates to a device for treating one or more layers of tissue, for example by simultaneously imparting perforations into the one or more layers. In particular, the invention relates to a device for treating one or more layers of the skin to elicit a healing response which produces a more desirable skin appearance.

### BACKGROUND OF THE INVENTION

Several undesirable conditions of the skin are commonly seen in dermatologic practice, which may be caused by age, exposure to the sun's ultraviolet rays, and other influences. For example, acne scars, burn scars, erythema, fine lines, wrinkles or other irregular conditions are undesirable. There are currently several ways to treat these conditions. For example, many topical medications are available such as retinoic acid, hydroxyquinones, alpha hydroxy acids and chemicals peels. These offer some improvement in skin texture and coloration, yet they are irritating to use and only offer mild improvement. More aggressive measures using dermabrasion, lasers and surgical scar revisions have also been used.

All of these methods are generally used to horizontally treat or remove one or more layers of tissue, so that entire layers of tissue are seared, cauterized or otherwise removed. Thus, all of these methods remove entire layers of skin or tissue, so that new layers form during healing. Cosmetic improvement is seen when the skin containing wrinkles or another undesirable mark is replaced by a new layer of skin.

However, all of these methods disrupt and completely remove the epidermis. The resulting open wounds require daily care to optimize wound healing. The open wounds also increase the risk of infection, which can lead to prolonged healing time and scarring. Procedures involving the complete removal of the epidermis are also painful and require general anesthesia. Also, due to the amount and type of tissue removal, one or two weeks of healing time and constant skin care are needed. Also, patients often experiences two to four months of having red sensitive skin.

Epidermal destruction and subsequent healing may also cause side effects including prolonged hypopigmentation, hyperpigmentation, ervthema and edema. Hyperpigmentation occurs frequently in darker skin types as a result of an inflammatory response of the skin. Hyperpigmentation results in the treated area of the subject's skin turning darker than the surrounding untreated skin. Hyperpigmentation can be slow to clear, sometimes taking up to a year to disappear. Hypopigmentation is attributable to damage to the melanin-producing cells in the skin. While generally transient, hypopigmentation can be permanent, and is cosmetically undesirable while it persists. Also, erythema or redness of the skin may be significant for weeks to months after the procedure, requiring the patients to wear conspicuous amounts of make-up.

Certain methods have been developed to treat one or more layers of skin without removing entire layers of tissue. One example of device which treats tissue in this manner is the FRAXEL infrared scanning laser. This device uses a scanner to direct a small 10-70 micron diameter laser beam across the tissue surface in order to create small vertical zones of coagulated tissue. One drawback with the FRAXEL laser is that the laser does not create perforations or vertical holes in the tissue, but only causes zones of coagulation. Applicant has discovered that it is desirable to create vertical perforations or ablation holes in tissue, as this prompts an even more aggressive wound healing response than is currently seen. When perforations are created, tissue layers surrounding the perforations are left untreated and contribute to tissue regeneration. Thus, the treated tissue can heal and regenerate from all edges of the perforation wound, not just from the tissue areas underneath the wound. Perforations allow for a faster regeneration and healing time. The use of perforations are also advantageous in that by leaving zones of untreated tissue in between the perforations, less scarring and/or pigmentations are visible. Thus, there is a need for a device and method which imparts actual perforations into the skin, thereby promoting more aggressive healing.

Another drawback with the FRAXEL laser and other devices is that they are manually operated by the medical provider. The quality of the treatment depends largely on the medical provider's skill in operating the device. With the FRAXEL laser, the precise number of coagulations in the tissue is entirely dependant upon the speed and number of passes the laser is moved along the skin by the operator. Thus, there is a lot of room for operator error and it is difficult to control or otherwise standardize the treatment. Control circuits have also been developed to monitor and track the speed of treatment. However, such circuits are often complicated and expensive. Therefore, there is a need for a device which allows for more standardized treatment and which reduces the chance of operator error. There is also a need for a device which is more simplified and less expensive than current devices.

Additionally, even if a simple knife, needle or other device is used to cause perforations in tissue, this would be a cumbersome process and would require that perforations be made one at a time. Again, the depth, width, and pattern of perforations would be subjected to the skill of the operator and would not be standardized. Thus, there is a need for a more standardized device which can impart perforations into tissue in a simultaneous and standardized manner. Also, when perforations are made mechanically rather than with the use of energy, excess bleeding takes place. Thus, there is also a need for a device which creates perforations without causing undue bleeding.

A yet another drawback to lasers and other prior devices is that they use deep penetrating laser light or other harmful energies which can cause ocular injury if used too close to the eyes. There are often many skin conditions which are present near the eyes which would benefit from aggressive treatments. Thus, it is desirable for a device which uses a form of energy that is safe when used close to the eyes and other delicate body parts. A device for skin treatment is known from US 6,277,116 B1. This device provides energy to the skin of a patient for shrinking collagen tissue in the dermis. The device includes a carrier and an array of needle electrodes on the carrier, which extend into the tissue to heat a dermal skin region.

### BRIEF SUMMARY OF THE INVENTION

Some examples disclose a device for creating a pattern of perforations in a tissue. The device comprises a treating surface configured to be positioned in contact with tissue adjacent one or more tissue planes and a plurality of electrodes extending outwardly from the treating surface and adapted for imparting simultaneous perforations into one or more tissue layers, wherein the electrodes are provided in a pattern to impart a corresponding pattern of perforations in the one or more tissue layers. The electrode pattern can be selected to create perforations which are between about 30 to about 100 microns in diameter, up to about 1000 microns deep and spaced apart by between about 50 to about 400 microns. The electrode pattern can also be selected to create a zone of coagulative tissue surrounding each perforation. In some cases, the zone of coagulative tissue has a length of between about about 5 microns to about 100 microns. The electrodes can also have a depth and width for providing perforations being no more than about 2 mm in depth and about 0.5 mm in width. Likewise, the electrodes can have a spacing for providing perforations spaced apart by no more than about 5 mm or less.

The device also includes an energy source coupled to the plurality of electrodes, the energy source configured to deliver energy selected from the group consisting of radio frequency, non-ionizing ultraviolet radiation, or microwave radiation. A control device may also be coupled to the energy source. In preferred cases, the electrodes are RF electrodes and are configured for receiving RF energy. The electrodes can be provided as a plurality of electrode pairs or even as an array of electrodes. In some cases, the electrodes are monopolar electrodes whereas in other cases the electrodes are bipolar electrodes. The electrodes can also be configured to provide power in the range of about 50 to about 200 watts per square centimeter and also configured to provide an energy of at least about 1 joule per square centimeter. In many cases, one or more sensors are coupled to the plurality of electrodes.

The treating surface of the treatment device can be selected from the group consisting of a flexible surface, contoured surface, rigid surface, horizontal surface, rolling surface, expandable surface and three-dimensional surface. In some cases, the treating surface is an expandable surface and is sized in an expanded state to conform to a surface of a tissue. In other cases, the treating surface is a horizontal surface and is applied to the one or more tissue planes in a stamping motion. In yet other cases, the treating surface is a rolling surface and is applied to the one or more tissue planes in a rolling motion.

In certain embodiments, a device is provided for creating a pattern of perforations in a tissue. The device includes an elongated member, a treating surface coupled to a distal end of the elongated member and configured to be positioned adjacent one or more tissue planes, and a plurality of monopolar RF electrodes extending outwardly from at least one surface of the treating surface and adapted for imparting simultaneous perforations into one or more tissue layers, wherein the electrodes are provided in a pattern to impart a corresponding pattern of perforations in the one or more tissue layers.

A method for creating a pattern of perforations in tissue is also provided. The method includes the steps of providing a device having a treating surface which includes a plurality of electrodes extending outwardly from the treating surface, wherein the plurality of electrodes are arranged in a desired pattern, placing the treating surface in contact with tissue adjacent to one or more tissue planes, and delivering energy to the electrodes to simultaneously impart perforations into one or more layers of the tissue, wherein the perforations correspond to the electrode pattern.

A kit for creating a pattern of perforations in tissue is also provided. The kit includes two or more devices, wherein each of the devices have either a differently sized treating surface, a differently shaped treating surface, a different treating surface type, a different electrode pattern, a different electrode width, a different electrode length or a different electrode spacing.

A method for treating human skin is also provided. The method includes identifying a target area of skin, providing a device adapted to simultaneously create a desired pattern of perforations into one or more layers of the target area of skin, and simultaneously perforating the target area to provide the desired pattern of perforations which elicit a healing response that produces a revitalized skin surface. The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

### BRIEF DESCRIPTION OF THE DRAWINGS

Figure 1 is a side view of the distal end of a treatment device in accordance with one embodiment of the invention.
Figure 2 is a side view of the distal end of a treatment device in accordance with another embodiment of the invention.
Figure 3 is a side view of the distal end of a treatment device in accordance with yet another embodiment of the invention.
Figure 4 is a front view of the distal end of a treatment device showing an electrode pattern in accordance with one embodiment of the invention.
Figure 5 is a front view of the distal end of a treatment device showing an electrode pattern in accordance with another embodiment of the invention.
Figure 6 is a front view of the distal end of a treatment device showing an electrode pattern in accordance with another embodiment of the invention.
Figure 7 is a front view of the distal end of a treatment device showing an electrode pattern in accordance with another embodiment of the invention.
Figure 8 is a cross-sectional view of a treated skin surface according to a prior art method.
Figure 9 is a cross-sectional view of a treated skin surface according to an embodiment of the invention.
Figure 10 is a top view of a treated skin surface according to an embodiment of the invention.

### DETAILED DESCRIPTION OF THE PREFERRED EMBODIMENTS

A device and method are provided for treating one or more tissue layers by providing a plurality of vertical perforations in a simultaneous fashion. The device does not remove entire layers of tissue, but rather creates perforations in one or more layers. These perforations create holes, which extend vertically into the tissue and across one or more layers. In some cases, the holes extend deep into the tissue across several layers. In other cases, the holes are shallow, and extend across only one or two superficial layers of tissue. As used herein, the term "perforation" means vertical areas of thermal damage or ablation to the tissue causing tissue necrosis.

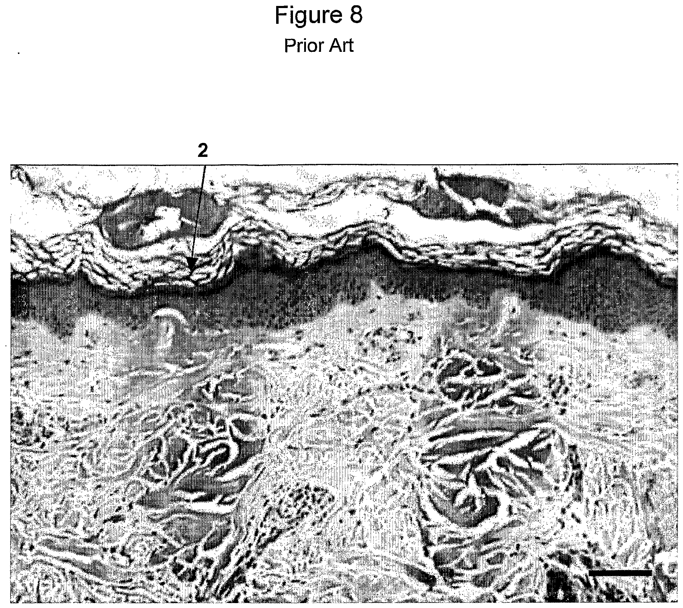

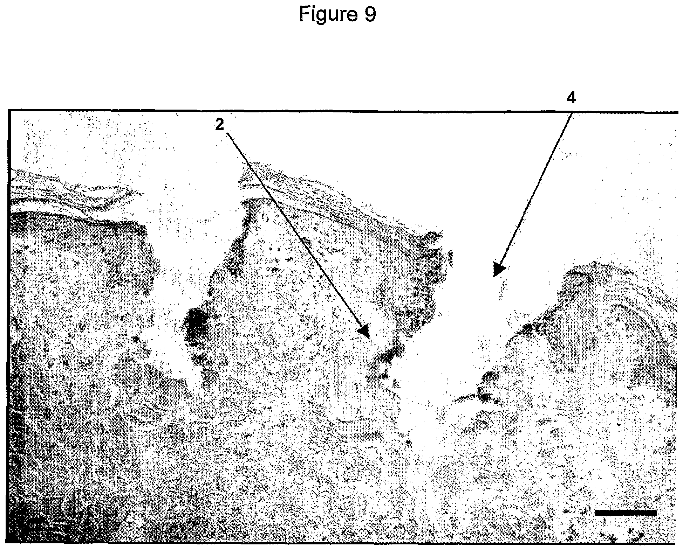

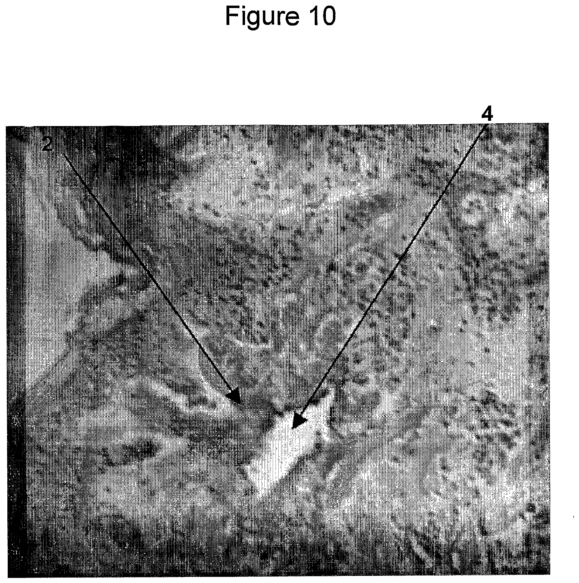

Figure 8 illustrates a skin area treated in accordance with a prior method. The skin area in Figure 8 has been treated using a FRAXEL infrared scanning laser. The laser causes coagulation, indicated at reference number 2. A perforation is not created at area 2, which is treated by the laser. On the other hand, Figure 9 illustrates a skin area treated in accordance with the invention. The treated area 4 is clearly a perforation, which extends vertically into the skin. Areas or zones of coagulation 2 can also be seen in the areas surrounding the perforation 4. Likewise, with reference to Figure 10, zones of coagulation are seen on areas of the skin surface surrounding the perforation 4. Generally, these zones extend into the tissue in all directions originating from the perforation site. These zones of coagulation are beneficial as coagulation leads to tissue regeneration.

The device and method also allow for controlled placement of perforations having a predetermined depth, width and degree of separation. Such perforations are so small that they cannot be seen with the naked eye. The treatment device is advantageous because it creates several perforations simultaneously with one or a few discharges of energy.

Many conditions can be treated by imparting a plurality of vertical perforations into tissue. In most cases, skin conditions are treated by creating perforations into the skin. For example, the device can be used on skin areas suffering from photo-aging or sun-damage. The device can also be used on skin areas exhibiting acne scars, burn scars, erythema, fine lines, wrinkles, irregular pigmentations, precancerous or cancerous lesions or other irregular conditions. The creation of perforations in the skin helps to revitalize or rejuvenate these irregular areas to make them more desirable in appearance. The zones of coagulation created in the tissue area surrounding each perforation also help to revitalize and rejuventate skin.

In other cases, the device and method are used to treat the uterus lining for excess menometorrhagia. In yet other cases, the device and method are used to treat conductive abnormalities of the heart. The heart could also be treated to treat conductive abnormalities by ablating multiple foci of aberrant electrical paths.

The treatment device and method provide several advantages. For example, the treatment allows for a more standardized and controlled method of treating tissue, since the perforations are provided in a pre-selected pattern. An operator uses the device to impart the pre-selected pattern of perforations into the skin and does not create each perforation individually, thereby reducing chances of operator error. The device also imparts perforations into the skin, rather than merely imparting coagulations into the skin. The creation of actual perforations leads to more aggressive healing responses. Additionally, the use of perforations into skin rather than removal of entire skin layers reduces the chances of scarring and promotes a faster healing time.

The treatment device includes a treating surface, which is adapted to be placed in direct contact with a tissue adjacent a tissue plane to be treated. The treating surface is generally positioned adjacent to a tissue plane of the tissue to be treated. One or more layers of the tissue in the tissue plane can be treated. In some cases, it is desirable to only treat the outermost layer of tissue, while leaving the deeper layers untreated. In other cases, it is desirable to treat the deeper layers of tissue as well. The tissue to be treated can include almost any tissue. In some cases, the tissue includes one or more layers of organ tissue. In other cases, the tissue includes one or more layers of skin.

In many tissues and/or organs, natural layers are present. Typically, organ layers include an inner mucosal layer, a submucosal layer, a muscularis layer and an outer serosal layer. For example, an esophagus includes a mucosal layer, a submucosal layer, and a muscularis layer. A uterus wall includes a mucosal layer (known as the endometrium), a fibromusular layer (known as the myometrium) and an outer serosal layer. In some cases, it is desirable to treat an innermost mucosal layer, while leaving the intermediate submucosal layer intact. In other cases, it is desirable to treat both mucosal and submucosal layers, while leaving the muscularis layer intact. Again, any type and number of layers can be treated with the invention.

Similarly, the skin includes natural layers. Human skin consists mainly of two layers: the top layer of skin known as the epidermis; and the layer beneath the epidermis known as the dermis. The dermis is primarily acellular and is composed of water, the protein collagen, and glycosaminoglycans. Collagen and glycosaminoglycans are constantly produced by fibroblasts, a type of connective tissue cell, degraded by enzymes. With aging, the amount of dermal collagen decreases and is replaced by the protein elastin. In addition, the remaining collagen tends to be chaotically oriented as compared to the more organized patterns found in youthful skin.

Glycosaminoglycans are very hydrophilic, and increased amounts of these carbohydrates are associated with the increased skin vigor found in youthful skin. One major difference between the smooth, supple skin of newborns and the drier, thinned skin of older individuals is the far greater relative amount of glycosaminoglycans found in newborn skin. The glycosaminoglycans found in newborns can bind up to 1000 times their weight in water. As the skin ages and the amount of glycosarninoglycans decreases, the skin may become less hydrated and lose some of the suppleness found in youth. The treatment device can be used to create perforations and zones of coagulation across both the epidermis and dermis to activate fibroblasts which deposit increased amounts of extracellular matrix constituents (i.e., collagen and glycosaminoglycans). These increases in extracellular matrix constituents are responsible for dermal skin rejuvenation.

The treating surface of the treatment device can be provided in any desired shape or configuration. In most cases, the treatment device is used to treat layers of the skin, and a treating surface is provided having a surface which conforms to the external part of the body wherein the skin is treated. However, the size and shape of the treating surface is variable and often depends on the surface area of tissue to be treated. For example, the treating surface can be provided as a horizontal surface, three-dimensional surface, rigid surface, curved surface, contoured surface, expandable surface or the like.

In certain embodiments, the treating surface is an expandable surface, e.g., an expandable balloon. Suitable expandable treating surfaces include but are not limited to a balloon, compliant balloon, balloon with a tapered geometry, basket, plurality of struts, an expandable member with a furled and an unfurled state, one or more springs, foam, bladder, backing material that expands to an expanded configuration when unrestrained, and the like. The expandable surface can be made of a variety of different materials, including but not limited to an electroconductive elastomer such as a mixture of polymer, elastomer, and electroconductive particles

The expandable surface can be made to expand to a fixed size or a variable size. In particular cases, the expandable surface in its expanded state has a diameter in the range of between about 0.5 mm to about 5 cm. In cases where the treatment device is placed inside of a hollow organ, it may be desirable to provide a treating surface which has a shape and size which expands to conform to the interior shape of an organ. The expandable surface can also be configured to stretch the hollow interior of an organ. This stretching of tissue often impedes blood flow into the treatment area.

In certain cases, especially wherein the treatment device is used inside of the body, the treating surface can be provided as a three-dimensional surface, which corresponds to the surface of a particular body organ. For example, the surface can conform to the interior space of an organ to treat a layer of tissue lining the interior space. The surface can also conform to an exterior surface of an organ to treat the exterior layer of tissue lining the exterior surface of the organ.

In some cases, the treatment device includes an elongated member or shaft that has a proximal end and a distal end. The elongated member is especially desirable when using the treatment device inside of the body. The treating surface is generally provided about the distal end and in some cases, the treating surface may be the distal end of the elongated member itself. In such cases, the distal end is configured as a surface adapted for contacting a desired tissue plane. In most cases, however, the distal end will be coupled to a separately provided treating surface. The treating surface can be bonded or otherwise attached to an area along the distal end. In cases where the treatment device is placed inside of the body to treat menometorrhagia, conductive abnormalities of the heart or other internal conditions, an operator manipulates the proximal end to cause the distal end to be inserted into a desired place in the body. The distal end can be inserted and positioned into the body in any of various ways known in the art and selected by the operator, including using endoscopical methods, surgical methods and other methods. The treatment device can also include steerable and directional control devices to aid the operator in positioning the distal end within the body.

When the treating surface is expandable and desired to be expanded inside of the body, it may be desirable to provide the member in a folded positioned and placed within a sheath during positioning of the distal end within the body. This prevents the treating surface from taking up too much space, so the distal end can be guided through narrower channels in the body. Once the distal end is positioned at a desired site in the body, the sheath can be removed, for example by retracting it along the shaft to expose the treating surface.

The treating surface generally includes a plurality of electrodes positioned about at least a portion of its circumference so that the electrodes come into contact with the tissue. The electrodes can be provided about the entire surface of the treating surface or about a portion of the surface. The areas of tissue in contact with the electrodes are those areas which are perforated.

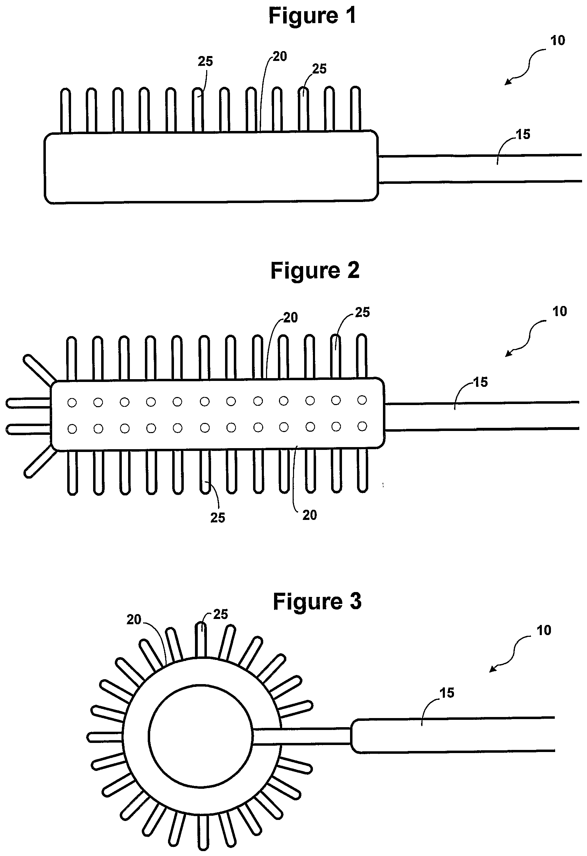

The electrodes are arranged in a pattern to create the desired pattern of perforation in tissue. The electrodes are preferably configured as spikes or pins which extend outwardly from the treating surface. For example, Figure 1 illustrates a treatment device 10 having a treating surface 20 connected to a distal end or shaft 15. A plurality of electrode pins 25 are positioned about a portion of the treating surface. When it is desired to apply the treating surface 20 of Figure 1, one uses a stamping motion and stamps the tissue layers with the treating surface 20.

Likewise, Figure 2 also illustrates a treatment device 10 having a plurality of electrode pins 25 positioned about a treating surface 20. However, the electrode pins in Figure 2 are positioned about substantially the entire surface of the treating surface whereas in Figure 1, the electrode pins are positioned only about one surface of the treating surface. Whereas the treating surfaces in the illustrated Figures have a flat or sheet-like shape, it should be understood that they can provided in any suitable shape and/or adapted to be expanded into any suitable shape.

Figure 3 illustrates a treatment device 10 having a rolling treating surface 20. Here the treating surface 20 can be rolled along a tissue plane, creating perforations along the way. When it is desired to apply the treating surface 20 of Figure 3, one uses a rolling motion and creates the perforations by rolling.

The electrodes are positioned on the treating surface in any manner to provide a desired pattern of perforations into the one or more tissue planes. Generally, the electrodes come into direct contact with the one or more tissue planes. So, the pattern of electrodes directly correspond to the pattern of perforations desired in the tissue. Any desired pattern or electrode arrangement is within the scope of the invention. In certain cases, the electrodes are patterned in order to create perforations being between about 30 to about 100 microns in diameter, up to about 1000 microns deep and spaced apart by between about 50 to about 400 microns. In other preferred cases, the electrodes have a depth and width for providing perforations being no more than about 2 mm in depth and about 0.5 mm in width. In yet other cases, the electrodes have a spacing for providing perforations spaced apart by no more than about 5 mm or less and more preferably no more than about 2 mm or less.

Zones of coagulation are also created in areas of tissue surrounding each perforation. The zones can extend from the perforation site into the tissue for a length ranging from about 5 microns to about 100 microns. The zone of coagulation can be varied by changes in energy and power levels .

It should be understood that not all of the electrodes present on a treating surface need to be of the same size. Also, the spacing between electrodes can be varied along the treating surface, to create certain desired affects. In some cases, the electrodes in the center of the treating surface are spaced more closely than the electrodes on the edges of the treating surface. This provides for a feathered effect, so that no sharp lines are seen at the areas where the treated skin meet the untreated skin.

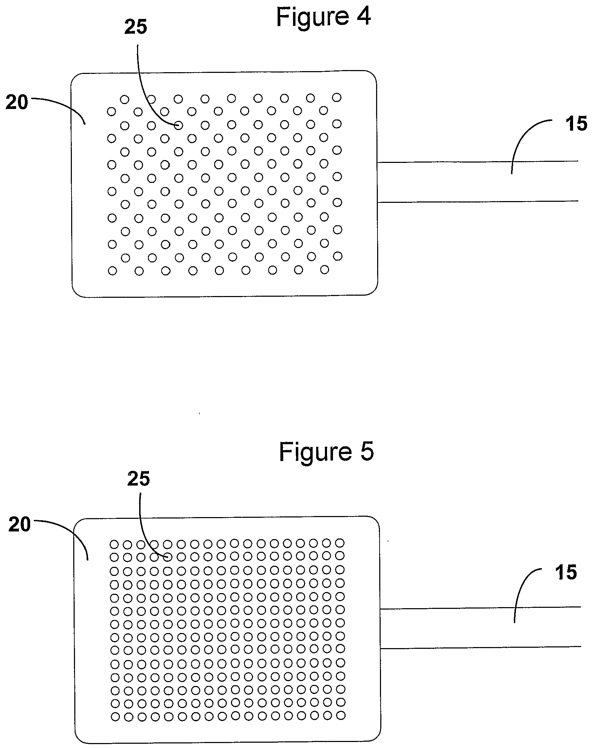

In some cases, the electrodes are arranged in an orderly pattern. Figures 4-7 illustrate electrodes 25 provided in various electrode patterns and positioned on a portion of the treating surface 20. Each of the Figures 4-7 illustrate an orderly pattern of electrodes. However, in some cases, a random electrode pattern may be desired. In further cases, a feathered pattern may be desired to reduce the lines seen between the treated tissue and untreated tissue. The length of the electrode arrangement on the treating surface can also be varied. In certain embodiments, the length of the plurality of electrodes is in the range of about 0.5 mm to about 5 cm.

The outer tips of each electrode are designed to be placed into contact with a tissue plane. Energy is delivered to the tip to provide the perforation of tissue. In some cases, when it is desirable to provide a deeper perforation, the electrode pins are placed all the way into the tissue, so that more than one layers of tissue are treated. When it is desirable to provide an even deeper perforation, the electrode pins can be providing having longer lengths, so that the tips are placed even deeper into the tissue layers. The length and width of the electrode pins are variable and can be chosen based on the desired treatment. Again, in preferred embodiments, the electrodes are provided having a length and width which creates perforations having a depth of no more than about 2 mm and a width of no more than about 0.5 mm.

The electrodes can also be spaced apart at a desired length so that the perforations in tissue will also be spaced apart at that length. Any length between electrodes is within the scope of the invention. In some cases, each of the electrodes on a treating surface are spaced apart evenly whereas in other cases, some electrodes on a treating surface are space apart at a smaller length than other electrodes. The spacing of the electrodes can be adjusted in order to provide any desired spacing of perforations. With reference to the Figures, the electrodes of Figure 3 have a larger spacing than the electrodes of Figure 4. In certain embodiments, electrodes are provided on a treating surface have a spacing of less than about 5 mm, and preferably less than about 2 mm.

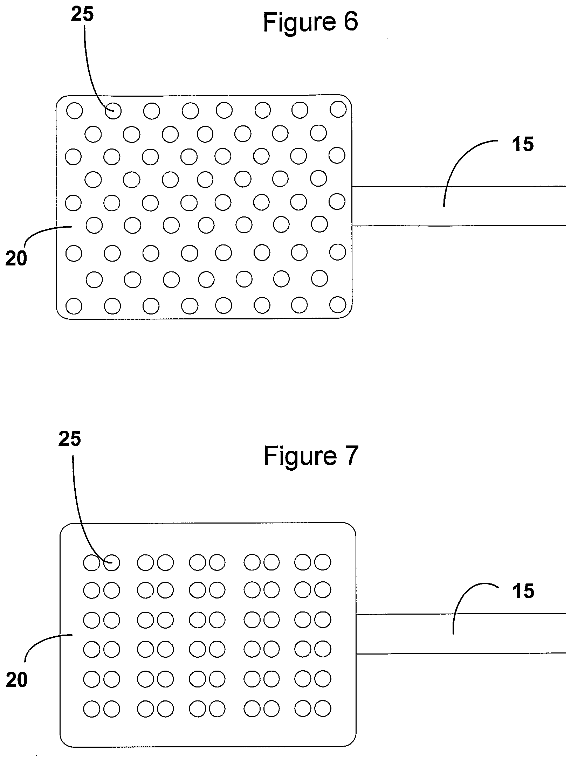

The width or diameter of each electrode pin can also be varied to provide a perforation having a desired width. For example, the electrodes of Figure 5 and 6 have a larger width than the electrodes of Figures 3 and 4. In most cases, the width of each electrode pin on a particular treating surface will be the same, although this is not necessary. Electrode pins having different widths can be provided. The width of the perforation created will be at least the width of the electrode, and in many cases larger. The width of the perforation created depends not only on the width of the electrode, but also the level of energy discharged form the electrode. When more energy is discharged from the electrode, a larger perforation width and depth is created. Additionally, the width of perforation may be tapered with depth.

The electrodes can also be arranged as an array of electrodes. In some cases, the electrode pairs are arranged as a contiguous sequence of arrays. In particular embodiments, the electrodes are provided as a contiguous sequence of arrays with a single common RF electrode along an entire length of the arrays. The electrodes can be also provided as a pattern of electrode pairs rather than as a pattern of individual electrodes. The electrodes in some cases are provides as an array of electrode pairs. In other cases, each electrode pair has an electrode which is divided into a sequence of selectable lengths. In further cases, each electrode in an electrode pair is parallel to an adjacent electrode. Figure 6 illustrates a pattern of electrode pairs. Doctors can choose a particular pattern based on the desired treatment needed for a tissue.

The electrodes can be either monopolar or bipolar electrodes. In some embodiments, the electrodes are monopolar electrodes. In a monopolar electrode arrangement, one electrode serves as a treating electrode and another electrode is provided as a return electrode. The return electrode generally has a much larger area than the treating electrode and is placed out of the treated area. In certain cases, the treating device of the invention is provided having a monopolar electrode arrangement, wherein the electrode pins extending outwardly from the treating surface serve as treating electrodes and a grounding pad is placed within the treating surface to serve as the return electrode. In embodiments wherein the electrodes are provided as individual electrodes rather than in pairs, monopolar electrodes are desirable.

In other embodiments, the electrodes are provided as a bipolar electrode arrangement. In bipolar electrode arrangements, both the positive and negative electrodes serve as treating electrodes. In some cases, the bipolar electrodes are biopolar axial interlaced finger electrodes. In embodiments wherein the electrodes are provided as electrode pairs, biopolar electrodes are desirable, with one electrode in the pair being a positive electrode and the other electrode being a negative electrode. The electrodes can be shaped in such a way that the middle portion of distal portion is enlarged in order to create a zone of injury that is greater at the distal end compared to the proximal end. The base of the treatment pad or treating surface and various portions of the proximal electrodes can also be insulated in order to limit the coagulation injury to the distal end of the electrode as well.

The treatment device also includes an energy source coupled to the electrodes for delivering energy to the electrodes. The energy delivery device can deliver a variety of different types of energy including but not limited to, radio frequency energy, non- ionizing ultraviolet radiation and microwave radiation. In some cases, the electrodes are configured to provide power in the range of about 50 to about 200 watts per square centimeter. In other cases, the electrodes are configured to provide an energy of at least about 1 joules per square centimeter.

In preferred embodiments, the electrodes are configured as RF electrodes and RF energy is delivered to the electrodes. RF energy is particularly desirable for creating perforations of tissue since it does not cause the entire area of tissue being treated to heat up extensively. Rather, RF energy can penetrate the body and be absorbed by deeper tissues without heating up the surrounding tissues. Thus, a boundary is created between the treated tissue and those tissues surrounding the treated tissue. RF energy is also desirable since it is safe to use on tissues in areas near sensitive body parts, e.g., areas of skin near the eyes.

The energy source is configured for powering the electrodes at levels appropriate to provide a desired diameter and depth of perforation of tissue. The energy source may be manually controlled by the user and be adapted to allow the user to select the appropriate treatment time and power setting to obtain a controlled depth and/or width of perforation. The energy source can also be coupled to a controller, which may be a digital or analog controller for use with the energy source, including but not limited to an RF source, or a computer with software. When the computer controller is used it can include a CPU coupled through a system bus. The system may include a keyboard, a disk drive, or other non-volatile memory system, a display and other peripherals known in the art. A program memory and a data memory can also be coupled to the bus. The energy source can be positioned at different positions in proximity to the treatment device.

For those treatment devices employing a variably shaped expandable member (e.g., that conforms to an oddly shaped organ or external body part), the desired power and energy settings can be scaled as needed so that each electrode delivers the same power and energy per unit area. These changes can be made either automatically or from user input to the RF power source. If different treatment depths are desired for one or more electrodes on the expandable member, the geometry of the some of the electrodes can be modified to create either a deeper or more superficial treatment region than other electrodes.

In some embodiments, one or more sensors can be positioned upon one or more electrode pins in order to monitor the temperature, depth or diameter of perforation, and the like. For example, in some cases, a temperature sensor is coupled to the electrodes. In other cases, a multiplexer is coupled to the electrodes. In yet other cases, a multiple-pin electrical connector is coupled to the electrodes.

In some embodiments, a surgical kit including differently sized treatment devices is provided, each device having a differently sized or shaped treating surface, different treating surface type, different electrode pattern, a different electrode size, diameter or length, and/or a different level of energy delivery. An operator can select various treatment devices which are best suited for a given application. Furthermore, when treating areas of the skin, the use of different devices can aid an operator in feathering the edges of the treatment zone, so that no sharp lines are seen at the areas where the treated skin meet the untreated skin. For example, the number of the perforations can be gradually decreased when moving from the treated skin to the untreated skin. This can be accomplished by using a device having a greater number of perforations on the treatment skin and then by supplementing this device with those having less perforations when moving from the treatment areas to the non-treated areas.

Methods for vertically treating one or more layers of tissue in a tissue plane are also provided. The method generally includes simultaneously imparting perforations into one or more tissue layers in a tissue plane. In a preferred embodiment, a method is provided for simultaneously imparting perforations into one or more layers of skin. In many cases, skin areas along the face, neck, chest and hands will be treated. The treatment area of the skin is first cleansed using a cleanser, for example a mild, gently abrasive skin cleanser. A topical may then be applied to the treatment area to numb the skin so that a patient will not feel and prickling or heat sensation during treatment. In some cases, the topical will be a lipid based topical anesthetic ointment. A treatment device according to any of the embodiments already described is provided and the treating surface is placed in contact with a desired area of the skin. The device is activated to create a plurality of perforations in the skin. In many cases, the same area of skin will be treated several times and usually at different time intervals.

In another embodiment, methods are provided for treating tissue inside of a body. The operator may first determine the length of the portion of the tissue needing treatment inside of the body by visual observation through an endoscope. The provider than selects a treatment device having an electrode pattern which is best suited for treating that portion of tissue. For example, when the tissue is a small patch of tissue on an esophagus, a treatment device having electrodes only on one surface may be desirable. On the other hand, when the issue is the interior lining of a hollow organ, the treatment device may include an expandable member which in an expanded state, conforms to and/or stretches the interior wall of the organ. Once the desired treatment device is in place, the energy source is activated to deliver energy to the electrodes. Following treatment, the medical provider may do an endoscopic evaluation of the treated areas.

### Example

A freshly exercised pig skin was obtained. A monopolar electrode array was placed upon the skin. The array consisted of two 0.3 mm length needles placed in a row 500 microns apart. The array was hooked up to an electrical generator. A power of 50 watts and an energy of 1 joule were delivered to the electrode array. A 4 mm punch biopsy was taken and process for routine light microscopy. Upon examination of the tissue, both the epidermis and dermis were perforated by two equivalent vertical perforations. The perforations were widest at the top (50 to 100 microns in diameter) and tapered to a point at a depth of 250-300 microns. Throughout the edge of the injury, a 10-50 micron zone of coagulation was also seen.

The foregoing description of a preferred embodiment of the invention has been presented for purposes of illustration and description. It is not intended to be exhaustive or to limit the invention to the precise forms disclosed. Obviously, many modifications and variations will be apparent to practitioners skilled in this art. It is intended that the scope of the invention be defined by the following claims.

## Claims

1. A device (10) for creating a pattern of perforations (4) in a skin tissue by delivering radio frequency energy to an array of needles, comprising
a treating surface (20) configured to be positioned in contact with skin tissue adjacent one or more tissue planes; and
a plurality of electrodes (25) arranged as the array of needles extending outwardly from the treating surface, the plurality of electrodes (25) being sized for imparting simultaneous perforations (4) into one or more tissue layers and adapted for delivering RF energy to at least one of the perforated tissue layers,
an energy source coupled to the plurality of electrodes (25), the energy source configured to deliver of radio frequency energy to the electrodes, and
a control device coupled to the energy source, wherein:
the array of needles are provided in a pattern to impart a corresponding pattern of perforations (4) in the one or more tissue layers, one or more portions of the electrodes (25) are insulated to limit energy delivery from the electrodes (25) to the one or more tissue layers, **characterised in that** the plurality of electrodes in the electrode pattern are configured to deliver RF energy to create a plurality of zones of coagulative tissue (2), each zone surrounding at least an uninsulated portion of each electrode (25) respectively, and wherein the energy source is configured to power the electrodes such that each zone of coagulative tissue (2) extends from a perforation site for a length of between about 5 microns to about 100 microns and wherein the length can be varied with a change in a level of the RF energy and
the plurality of electrodes in the electrode pattern are spaced to leave untreated tissues surrounding each zone of coagulative tissue (2).

2. The device (10) of claim 1,
wherein at least two of the plurality of electrodes (25) in the electrode pattern are spaced apart by 5mm or less.

3. The device (10) of claim 1,
wherein the electrode pattern is selected to create perforations (4) which are between about 30 to about 100 microns in diameter, up to about 1000 microns deep and spaced apart by between about 50 to about 400 microns.

4. The device (10) of claim 1,
wherein the plurality of electrodes (25) have a depth and width for providing perforations (4) being no more than about 2 mm in depth and about 0.5 mm in width.

5. The device (10) of claim 1,
wherein the plurality of electrodes (25) are provided as a plurality of electrode pairs.

6. The device (10) of claim 1,
wherein the plurality of electrodes (25) are monopolar electrodes.

7. The device (10) of claim 1,
wherein the plurality of electrodes (25) are bipolar electrodes.

8. The device (10) of claim 1,
wherein the treating surface (20) is selected from the group consisting of a flexible surface, contoured surface, rigid surface, horizontal surface, rolling surface, expandable surface and three-dimensional surface.

9. The device (10) of claim 8,
wherein the treating surface (20) is an expandable surface and is sized in an expanded state to conform to a surface of a tissue.

10. The device (10) of claim 8,
wherein the treating surface (20) is a horizontal surface and is applied to the one or more tissue planes in a stamping motion.

11. The device (10) of claim 8,
wherein the treating surface (20) is a rolling surface and is applied to the one or more tissue planes in a rolling motion.

12. The device (10) of any one of the preceding claims,
wherein the electrodes (25) are configured to provide power in the range of about 50 to about 200 watts per square centimetre.

13. The device (10) of any one of the preceding claims,
wherein the electrodes (25) are configured to provide an energy of at least about 1 joule per square centimetre.

14. The device (10) of any one of the preceding claims, further comprising
one or more sensors coupled to the plurality of electrodes (25).

15. The device (10) of claim 1,
wherein the electrode pattern is selected to create the perforations (4) which are spaced apart by 2mm or less.

## Patentansprüche

1. Ein Gerät (10) zur Erzeugung eines Perforationsmusters (4) in einem Hautgewebe durch Zuführung von Hochfrequenzenergie zu einem Array von Nadeln, umfassend:
eine Behandlungsoberfläche (20), welche ausgebildet ist, in Kontakt mit Hautgewebe positioniert zu werden, benachbart zu einer oder mehreren Gewebeebenen; und
eine Vielzahl von Elektroden (25), welche als das Array von Nadeln angeordnet sind, und welche sich von der Behandlungsoberfläche nach außen erstrecken, wobei die Vielzahl von Elektroden (25) so dimensioniert sind, gleichzeitige Perforationen (4) in einer oder mehreren Gewebelagen einzuprägen und welche angepasst sind, Hochfrequenzenergie zu zumindest einer der perforierten Gewebelagen zuzuführen,
eine Energiequelle, welche mit der Vielzahl von Elektroden (25) gekoppelt ist, wobei die Energiequelle ausgebildet ist, Hochfrequenzenergie zu den Elektroden zuzuführen, und
ein Steuerungsgerät, welches mit der Energiequelle gekoppelt ist, wobei:
das Array von Nadeln in einem Muster vorgesehen ist, um ein entsprechendes Muster von Perforationen (4) in die eine oder mehrere Gewebeebenen einzuprägen, eine oder mehrere Abschnitte der Elektroden (25) isoliert sind, um die Energiezufuhr von den Elektroden (25) zu der einen oder mehreren Gewebelagen zu begrenzen,
**dadurch gekennzeichnet, dass**
die Vielzahl von Elektroden in dem Elektrodenmuster ausgebildet sind, Hochfrequenzenergie zuzuführen, um eine Vielzahl von Zonen von koaguliertem Gewebe (2) zu erzeugen, wobei jede der Zonen jeweils zumindest eine der nicht isolierten Abschnitte von jedem der Elektroden (25) umgibt, und wobei die Energiequelle ausgebildet ist, die Elektroden anzuregen, so dass jede Zone des koagulierten Gewebes (2) sich von einer Perforationsstelle um eine Länge zwischen ungefähr 5 µm bis ungefähr 100 µm erstreckt, und wobei die Länge variiert werden kann mit einer Veränderung in einer Höhe der Hochfrequenzenergie, und
die Vielzahl von Elektroden im Elektrodenmuster voneinander beabstandet sind, um unbehandelte Gewebeteile zu belassen, welche jede der Zonen koagulierten Gewebes (2) umgeben.

2. Das Gerät (10) gemäß Anspruch 1,
wobei zumindest zwei der Vielzahl an Elektroden (25) im Elektrodenmuster um 5 mm oder weniger voneinander beabstandet sind.

3. Das Gerät (10) gemäß Anspruch 1,
wobei das Elektrodenmuster ausgewählt ist, Perforationen (4) zu erzeugen, welche einen Durchmesser zwischen ungefähr 30 µm und 100 µm aufweisen, bis zu ungefähr 1000 µm tief sind und zwischen ungefähr 50 µm bis ungefähr 400 µm voneinander beabstandet sind.

4. Das Gerät (10) gemäß Anspruch 1,
wobei die Vielzahl von Elektroden (25) eine Tiefe und Breite haben, um Perforationen (4) bereitzustellen, welche eine Tiefe von nicht mehr als 2 mm aufweisen und eine Breite von ungefähr 0,5 mm.

5. Das Gerät (10) gemäß Anspruch 1,
wobei die Vielzahl von Elektroden (25) als eine Vielzahl von Elektrodenpaaren vorgesehen sind.

6. Das Gerät (10) gemäß Anspruch 1,
wobei die Vielzahl von Elektroden (25) monopolare Elektroden sind.

7. Das Gerät (10) gemäß Anspruch 1,
wobei die Vielzahl von Elektroden (25) bipolare Elektroden sind.

8. Das Gerät (10) gemäß Anspruch 1,
wobei die Behandlungsoberfläche (20) ausgewählt ist von der Gruppe, welche aus Folgendem besteht: eine flexible Oberfläche, eine konturierte Oberfläche, eine steife Oberfläche, eine horizontale Oberfläche, eine eingerollte Oberfläche, eine expandierbare Oberfläche und eine dreidimensionale Oberfläche.

9. Das Gerät (10) gemäß Anspruch 8,
wobei die Behandlungsoberfläche (20) eine expandierbare Oberfläche ist und in einem expandierten Zustand so dimensioniert ist, dass diese einer Oberfläche eines Gewebes entspricht.

10. Das Gerät (10) gemäß Anspruch 8,
wobei die Behandlungsoberfläche (20) eine horizontale Oberfläche ist, welche auf die eine oder mehreren Gewebeebenen durch eine Stempelbewegung angewandt wird.

11. Das Gerät (10) gemäß Anspruch 8,
wobei die Behandlungsoberfläche (20) eine eingerollte Oberfläche ist, welche auf die eine oder mehrere Gewebeebenen in einer rollenden Bewegung angewandt wird.

12. Das Gerät (10) gemäß einem der vorangehenden Ansprüche,
wobei die Elektroden (25) ausgebildet sind, Leistung ihm Bereich von ungefähr 50 bis ungefähr 200 Watt pro Quadratzentimeter bereitzustellen.

13. Das Gerät (10) gemäß einem der vorhergehenden Ansprüche,
wobei die Elektroden (25) ausgebildet sind, eine Energie von zumindest ungefähr 1 Joule pro Quadratzentimeter bereitstellen.

14. Das Gerät (10) gemäß einem der vorangehenden Ansprüche, ferner umfassend eine oder mehrere Sensoren, welche mit der Vielzahl von Elektroden (25) gekoppelt sind.

15. Das Gerät (10) gemäß Anspruch 1,
wobei das Elektrodenmuster ausgewählt ist, die Perforationen (4) so zu erzeugen, dass diese um 2 Millimeter oder weniger voneinander beabstandet sind.

## Revendications

1. Un dispositif (10) pour créer un motif de perforations (4) dans un tissu cutané en délivrant de l'énergie radiofréquence à un réseau d'aiguilles, comprenant
une surface de traitement (20) configurée pour être positionnée en contact avec le tissu cutané de façon adjacente à un ou plusieurs plans du tissu ; et
une pluralité d'électrodes (25) disposées en tant que ledit réseau d'aiguilles, s'étendant vers l'extérieur à partir de la surface de traitement, la pluralité d'électrodes (25) étant dimensionnée pour aménager des perforations simultanées (4) dans une ou plusieurs couches de tissu et adaptée pour délivrer de l'énergie RF à au moins l'une des couches de tissu perforées,
une source d'énergie reliée à la pluralité d'électrodes (25), la source d'énergie étant configurée pour délivrer de l'énergie radiofréquence aux électrodes, et
un dispositif de commande relié à la source d'énergie,
le réseau d'aiguilles étant conçu selon un motif de façon à aménager un motif de perforations (4) correspondant dans la ou lesdites plusieurs couches de tissu, une ou plusieurs parties des électrodes (25) étant isolées pour limiter la distribution d'énergie depuis les électrodes (25) à ladite ou plusieurs couches de tissu,
**caractérisé en ce que**
la pluralité d'électrodes dans le motif d'électrodes est configurée pour délivrer de l'énergie RF afin de créer une pluralité de zones de tissu coagulatif (2), chaque zone entourant au moins une partie non isolée de chaque électrode (25) respective, et la source d'énergie étant configurée pour alimenter les électrodes de sorte que chaque zone de tissu coagulatif (2) s'étend depuis un site de perforation sur une longueur comprise entre environ 5 microns et environ 100 microns et la longueur pouvant varier avec un changement du niveau de l'énergie RF, et
les électrodes de la pluralité d'électrodes dans le motif d'électrodes étant espacées pour laisser des tissus non traités entourant chaque zone de tissu coagulatif (2).

2. Le dispositif (10) selon la revendication 1,
dans lequel au moins deux électrodes de la pluralité d'électrodes (25) dans le motif d'électrodes sont espacées de 5mm ou moins.

3. Le dispositif (10) selon la revendication 1,
dans lequel le motif d'électrode est sélectionné de façon à créer des perforations (4) qui ont entre environ 30 et environ 100 microns de diamètre, et jusqu'à environ 1000 microns de profondeur, et espacées d'environ 50 à environ 400 microns.

4. Le dispositif (10) selon la revendication 1,
dans lequel la pluralité d'électrodes (25) a une profondeur et une largeur permettant de fournir des perforations (4) qui n'ont pas plus d'environ 2 mm de profondeur et environ 0,5 mm de largeur.

5. Le dispositif (10) selon la revendication 1,
dans lequel la pluralité d'électrodes (25) est conçue sous la forme d'une pluralité de paires d'électrodes.

6. Le dispositif (10) selon la revendication 1,
dans lequel les électrodes de la pluralité d'électrodes (25) sont des électrodes monopolaires.

7. Le dispositif (10) selon la revendication 1,
dans lequel les électrodes de la pluralité d'électrodes (25) sont des électrodes bipolaires.

8. Le dispositif (10) selon la revendication 1,
dans lequel la surface de traitement (20) est choisie dans le groupe constitué par une surface flexible, une surface profilée, une surface rigide, une surface horizontale, une surface de roulement, une surface expansible et une surface tridimensionnelle.

9. Le dispositif (10) selon la revendication 8,
dans lequel la surface de traitement (20) est une surface expansible et est dimensionnée dans un état expansé pour se conformer à une surface d'un tissu.

10. Le dispositif (10) selon la revendication 8,
dans lequel la surface de traitement (20) est une surface horizontale et est appliquée sur le ou les plans de tissu selon un mouvement de tamponnage.

11. Le dispositif (10) selon la revendication 8,
dans lequel la surface de traitement (20) est une surface de roulement et est appliquée sur le ou les plans de tissu selon un mouvement de roulement.

12. Le dispositif (10) selon l'une quelconque des revendications précédentes,
dans lequel les électrodes (25) sont configurées pour fournir de l'énergie dans l'intervalle allant d'environ 50 à environ 200 watts par centimètre carré.

13. Le dispositif (10) selon l'une quelconque des revendications précédentes,
dans lequel les électrodes (25) sont configurées pour fournir une énergie d'au moins environ 1 joule par centimètre carré.

14. Le dispositif (10) selon l'une quelconque des revendications précédentes, comprenant en outre un ou plusieurs capteurs reliés à la pluralité d'électrodes (25).

15. Le dispositif (10) selon la revendication 1,
dans lequel le motif d'électrode est sélectionné pour créer les perforations (4) qui sont espacées de 2 mm ou moins.
